(19) Europäisches Patentamt
European Patent Office
Office européen des brevets

(11) Veröffentlichungsnummer: **0 221 516**
**A1**

(12) # EUROPÄISCHE PATENTANMELDUNG

(21) Anmeldenummer: **86115115.7**

(22) Anmeldetag: **31.10.86**

(51) Int. Cl.4: **C07D 215/22**, C07D 405/12, C07D 401/12, A01N 43/42

(30) Priorität: **06.11.85 DE 3539571**

(43) Veröffentlichungstag der Anmeldung:
**13.05.87 Patentblatt 87/20**

(84) Benannte Vertragsstaaten:
**AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Harre, Michael, Dr.**
**Fröhaufstrasse 8**
**D-1000 Berlin 41(DE)**
Erfinder: **Arndt, Friedrich, Dr.**
**Mühlenfeldstrasse 10**
**D-1000 Berlin 28(DE)**

(54) **7-(Aryloxy)-2-chinolyloxy-alkansäurederivate, Verfahren zur Herstellung dieser Verbindungen sowie diese enthaltende Mittel mit herbizider Wirkung.**

(57) Es werden neue 7-(Aryloxy)-2-chinolyloxy-alkancarbonsäurederivate der allgemeinen Formel I

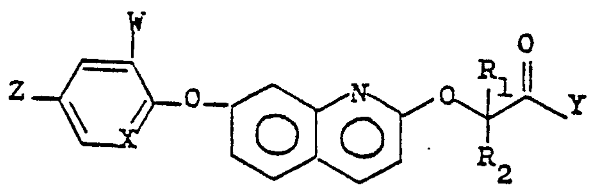

(I),

in welcher die Substituenten die im Anspruch 1 angegebene Bedeutung haben und ein Verfahren zu ihrer Herstellung beschrieben. Die erfindungsgemäßen Verbindungen zeigen eine herbizide Wirkung und sind zur Bekämpfung dikotyler und monokotyler Pflanzenarten in Nutzpflanzenkulturen geeignet.

EP 0 221 516 A1

# 7-(Aryloxy)-2-chinolyloxy-alkansäurederivate, Verfahren zur Herstellung dieser Verbindungen sowie diese enthaltende Mittel mit herbizider Wirkung

Die Erfindung betrifft neue racemische und optisch aktive 7-(Aryloxy)-2-chinolyloxy-alkancarbonsäurederivate, Verfahren zu ihrer Herstellung sowie Mittel mit herbizider Wirkung gemäß den Ansprüchen.

Konstitutionsanaloge Wirkstoffe auf Basis von Phenoxyalkancarbonsäurederivaten (JP-OS 92.369/80) und auf Basis von Phenoxybenzoesäurederivaten (US-PS 3.979.437) sind bereits bekannt.

Aufgabe der vorliegenden Erfindung ist die Schaffung neuer Wirkstoffe und eines diese Wirkstoffe enthaltendes herbiziden Mittels mit überlegenen Eigenschaften.

Diese Aufgabe wird erfindungsgemäß gelöst durch 7-(Aryloxy)-2-chinolyloxy-alkancarbonsäurederivate der allgemeinen Formel I

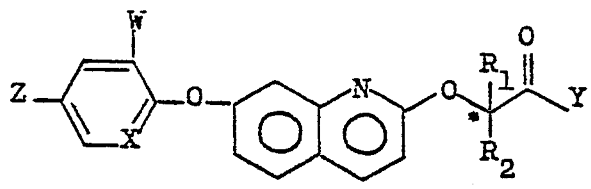

(I),

in welcher

Z und W unabhängig voneinander Wasserstoff, Halogen, einen $C_{1-4}$-Alkylrest, einen Trihalogenmethylrest oder eine Cyanogruppe bezeichnen,

X eine CH-Gruppe, eine C-Halogen-Gruppe oder ein Stickstoffatom bedeutet,

$R_1$ und $R_2$ unabhängig voneinander Wasserstoff oder eine $C_{1-4}$-Alkylgruppe darstellen und

Y die Gruppe

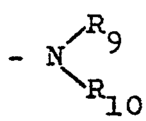

bedeutet,

wobei $R_9$ und $R_{10}$ gleich oder verschieden sind und jeweils Wasserstoff, einen $C_{1-18}$-Alkylrest, einen substituierten $C_{1-18}$-Alkylrest, einen $C_{2-8}$-Alkenyl-oder Alkinylrest, einen gegebenenfalls substituierten Aryl-$C_{1-3}$-Alkylrest, einen gegebenenfalls substituierten $C_{3-8}$-cycloaliphatischen Kohlenwasserstoffrest, einen gegebenenfalls substituierten $C_{3-8}$-Cycloalkyl-$C_{1-3}$-alkylrest, einen gegebenenfalls ein-oder mehrfach durch $C_{1-6}$-Alkyl und/oder Halogen und/oder $C_{1-6}$-Alkoxy und/oder die Nitrogruppe und/oder die Trifluormethylgruppe substituierten aromatischen Kohlenwasserstoffrest oder $R_9$ und $R_{10}$ gemeinsam mit dem N-Atom die Morpholino-, Piperidino-oder Pyrrolidinogruppe darstellen oder

Y den Rest $UR_3$ bezeichnet, wobei U ein Sauerstoff-oder Schwefelatom bedeutet und

$R_3$ einen $C_{1-18}$-Alkylrest, der gegebenenfalls durch Halogen oder Cyano substituiert oder durch Sauerstoff oder Schwefel ein-oder mehrfach unterbrochen ist, einen $C_{3-12}$-Alkenyl-, $C_{3-12}$ -Alkinyl oder $C_{3-12}$-Cycloalkylrest, einen Phenyl-oder Benzylrest, der unsubstituiert oder durch Halogen, $C_{1-4}$-Alkyl, $C_{1-4}$-Alkoxy, $C_{1-4}$-Alkylthio, Nitro, Cyano oder Trifluormethyl ein-oder mehrfach substituiert ist oder einen 5-6 gliedrigen heterocyclischen Rest darstellt;

Wasserstoff, Lithium, Natrium oder Kalium oder ein Äquivalent von Zink, Mangan , Calcium, Magnesium oder Barium oder ein Ammonium-Ion der Formel

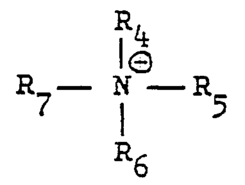

bedeutet, wobei
$R_4$, $R_5$, $R_6$ und $R_7$ unabhängig voneinander Wasserstoff, Alkyl mit bis zu 6 Kohlenstoffatomen oder halogen-, hydroxy-oder alkoxy-substituiertes Alkyl mit bis zu 6 Kohlenstoffatomen oder eine Carbalkoxyalkyl-Gruppe -$(CH_2)_n$ $COOR_8$ mit n = 1-4 und R = $C_{1-4}$-Alkyl darstellen.

Die Verbindungen der Formel I weisen ein asymmetrisches *C-Atom auf, wenn die beiden Substituenten $R_2$ und $R_1$ nicht identisch sind. Somit betrifft die Erfindung die einzelnen optischen Antipoden sowie die racemischen Mischungen.

In den Substituenten in Formel I können die Alkyl-, Alkenyl-oder Alkinylreste geradkettig oder verzweigt sein. Als Halogenatome sind bevorzugt Chlor oder Brom. Beispiele für fünf-und sechsgliedrige heterocyclische Reste sind Furan, Pyran, Pyrrol, Pyrrolidin, Pyridin, Piperidin, Pyrazon, Pyrazolidin, Pyrimidin, Oxazolidin, Oxazol, Morpholin, Thiazol und Thiazolidin.

Von den erfindungsgemäßen Verbindungen zeichnen sich in der Wirkung insbesondere diejenigen aus, bei denen in der allgemeinen Formel I
Z und W unabhängig voneinander ein Fluor-, Chlor-, Brom-oder Wasserstoffatom, eine Cyano-, eine Trifluormethyl-, eine Trichlormethylgruppe, eine Methyl-, Ethyl-oder Isopropylgruppe bedeuten,
X eine CH-Gruppe, eine C-Fluor-, eine C-Chlor-, eine C-Brom-Gruppierung oder ein Stickstoffatom bedeutet,
$R_1$ und $R_2$ unabhängig voneinander Wasserstoff oder einen gegebenenfalls durch Chlor oder Brom ein-oder mehrfach substituiertes Methyl, Ethyl, n-Propyl, n-Butyl, iso-Propyl sowie Fluormethyl und Difluormethyl bedeuten,
Y den Rest $UR_3$ bezeichnet, wobei U ein Sauerstoff-oder Schwefelatom ist und
$R_3$ Wasserstoff, Methyl, Ethyl, Propyl, Isopropyl, Butyl, Allyl, Propargyl, Cyclohexyl, Pentyl, Hexyl, Dodecyl, Methoxyethyl, Ethoxyethyl, Benzyl, 4-Chlorbenzyl, 4-Nitrobenzyl, Phenyl, 4-Chlorphenyl, 4-Nitrophenyl, 3-Phenoxybenzyl oder Lithium, Natrium oder Kalium oder ein Äquivalent von Zink, Mangan, Calcium, Magnesium oder Barium oder Ethylammoniumion, Diethylammoniumion, Triethylammoniumion, Tetraethylammoniumion oder Tetrabutylammoniumion bedeutet
oder
Y die Gruppe

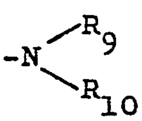

darstellt, wobei $R_9$ und $R_{10}$ unabhängig voneinander Wasserstoff, Methyl, Ethyl, Propyl, Isopropyl, n-Butyl, tert.-Butyl, 2,2-Dimethyl-1-propyl, n-Heptyl, n-Nonyl, n-Undecyl, n-Octadecyl, 3-Methylbutyl, 4-Methyl-2-pentyl, Isobutyl, 3,3-Dimethylbutyl, 2-Chlorethyl, 3-Chlorpropyl, 3-Brompropyl, 2-Bromethyl, 1-Phenoxy-2-propyl, Tetrahydrofurfuryl, Ethoxycarbonylmethyl, Cyanmethyl, 2,2-Dimethoxyethyl oder 2-Ethoxyethyl, Cyclohexylmethyl, 4-Cyanocyclohexylmethyl, 4-Hydroxymethylcyclohexylmethyl, Cycloheptylmethyl oder Cyclooctylmethyl oder Cyclopropylmethyl, 2-Propenyl, 2-Butenyl, 2-Methyl-2-propenyl, 2-Propinyl oder 3-Ethyl-1-pentin-3-yl, Benzyl, 4-Chlorbenzyl, 3-Chlorbenzyl, 2-Chlorbenzyl, 4-Fluorbenzyl, 3-Fluorbenzyl, 2-Fluorbenzyl, 4-Methylbenzyl, 3-Methylbenzyl, 2-Methylbenzyl, 3,4-Methylendioxybenzyl, 2,4-Dichlorbenzyl, 3,4-Dichlorbenzyl, 4-Methoxybenzyl, 3-Methoxybenzyl, 2-Methoxybenzyl, 2-Pyridylmethyl, 3-Pyridylmethyl, 4-Pyridylmethyl, α, α-Dimethylbenzyl, 1-Phenylethyl, 2-Phenylethyl, 1,2-Diphenylethyl, 2,2-Diphenylethyl, 4-Fluor-α-methylbenzyl, 3-Phenylpropyl, Cyclopropyl, Cyclopentyl, Cyclohexyl, 2-Methylcyclohexyl, 3-Methylcyclohexyl, 4-Methylcyclohexyl, 1-Ethinylcyclohexyl, Cycloheptyl oder Cyclooctyl, Phenyl, 3-Chlorphenyl, 2-Chlorphenyl, 4-Chlorphenyl, 2,4-Dichlorphenyl, 4-Fluorphenyl, 2-Methylphenyl, 3-Methylphenyl, 4-Methylphenyl, 1-Naphthyl, 2-Methoxyphenyl, 3-Methoxyphenyl oder 4-Nitrophenyl bedeuten.

Des weiteren sind aufgrund ihrer herbiziden Wirkung solche Verbindungen der Formel I bevorzugt, bei denen am asymmetrischen C-Atom die R-Konfiguration vorliegt.

Die erfindungsgemäßen Verbindungen lassen sich herstellen, indem man

(a) Verbindungen der allgemeinen Formel II

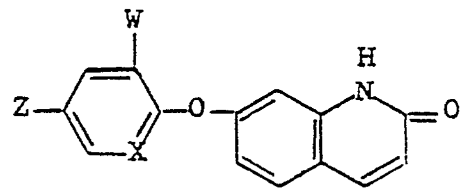

(II),

in der Z, W und X die bei Formel I angegebene Bedeutung haben,
mit einer Verbindung der allgemeinen Formel III

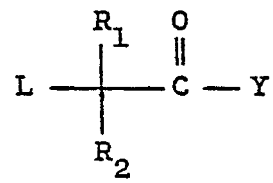

(III),

in der $R_1$, $R_2$ und Y die in Formel I angegebene Bedeutung haben und L für ein Halogenatom, den Mesyl- oder Tosyl-Rest steht, umsetzt
oder

(b) eine Verbindung der allgemeinen Formel IV

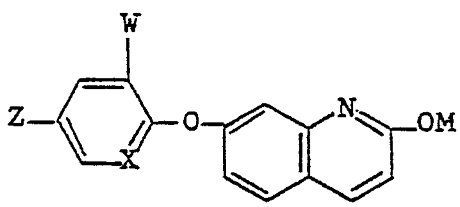

(IV),

in der Z, W und X die in Formel I angegebene Bedeutung haben und M ein Alkalimetall bezeichnet, mit einer Verbindung der Formel III

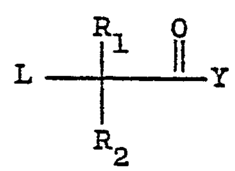

(III),

in der $R_1$, $R_2$, L und Y die oben angegebene Bedeutung haben, umsetzt,
oder

(c) eine Verbindung der allgemeinen Formel V

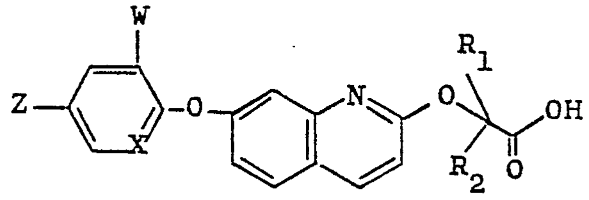

(V),

in der Z, W, X, $R_1$ und $R_2$ die in Formel I angegebene Bedeutung haben, mit einem Reagenz umsetzt, welches die Carboxylgruppe gegenüber einem nucleophilen Angriff aktiviert, vorzugsweise mit Thionylchlorid, Dicyclohexylcarbodiimid oder Carbonyldiimidazol, und das so erhaltene aktivierte Carbonsäurederivat mit einer Verbindung der Formel VI

H-Y (VI),

in der Y die in Formel I angegebene Bedeutung hat, reagieren läßt.

Die Reaktionsvariante (a) wird vorzugsweise in Gegenwart eines Verdünnungsmittels durchgeführt. Zu diesem Zweck können alle solchen Lösungsmittel verwendet werden, die gegenüber den Reaktanden inert sind. Beispiele für solche Lösungsmittel bzw. Verdünnungsmittel sind Wasser, aliphatische, alicyclische und aromatische Kohlenwasserstoffe, die jeweils gegebenenfalls chloriert sein können, wie z.B. Hexan, Cyclohexan, Petrolether, Ligroin, Benzol, Toluol, Xylol, Methylenchlorid, Chloroform, Kohlenwasserstofftetrachlorid, Ethylenchlorid, Trichloroethylen, Chlorbenzol; Ether wie z.B. Diethylether, Methylethylether, Methyl-t-butylether, Diisopropylether, Dibutylether, Dioxan und Tetrahydrofuran; Ketone wie z.B. Aceton, Methylethylketon, Methylisopropylketon und Methylisobutylketon; Ntrile wie z.B. Acetonitril und Propionitril; Alkohole wie z.B. Methanol, Ethanol, Isopropanol, Butanol und Ethylenglycol; Ester wie z.B. Ethylacetat und Amylacetat; Säureamide wie z.B. Dimethylformamid und Dimethylacetamid; Sulfone und Sulfoxide wie z.B. Dimethylsulfoxid und Sulfolan sowie Basen wie z.B. Pyridin.

Die Reaktionsvariante (a) wird weiterhin vorzugsweise in Gegenwart eines säurebindenden Mittels durchgeführt. Als Beispiele für solche säurebindenden Mittel seien Hydroxide, Carbonate, Hydrogencarbonate und Alkoholate von Alkalimetallen und tertiäre Amine wie Triethylamin und Pyridin genannt.

Die Reaktionsvariante (a) kann innerhalb eines breiten Temperaturbereichs durchgeführt werden. Im allgemeinen wird sie bei einer Temperatur zwischen -20 °C und dem Siedepunkt des Reaktionsgemisches, vorzugsweise bei 0 °C bis 150 °C, durchgeführt.

Die Reactionsvariante (a) wird vorzugsweise unter dem Druck der Umgebung durchgeführt, wenngleich sie auch bei erhöhtem oder vermindertem Druck durchgeführt werden könnte.

Die Reaktionsvariante (b) wird unter den gleichen Reaktionsbedingungen in bezug auf Temperatur und Druck durchgeführt, wie sie für die Reaktionsvariante (a) beschrieben wurden. Bei der Durchführung der Reactionsvariante (b) wird eines der für die Reaktionsvariante (a) beschriebenen Lösungsmittel bzw. Verdünnungsmittel verwendet, wobei allerdings vorher die Verbindung der allgemeinen Formel IV durch Behandeln der Verbindung der allgemeinen Formel II mit einer starken Base wie z.B. Natriumhydrid oder Kaliumhydrid hergestellt wird.

Die Reaktionsvariante (c) wird unter den gleichen Reaktionsbedingungen in bezug auf Temperatur, Druck und Lösungsmittel bzw. Verdünnungsmittel durchgeführt wie die Reaktionsvariante (a).

Die Gegenwart eines Reaktionskatalysators kann von Vorteil sein. Als Katalysatoren sind Kaliumjodid und Oniumverbindungen geeignet, wie quarternäre Ammonium-, Phosphonium-und Arsoniumverbindungen sowie Sulfoniumverbindungen. Ebenfalls geeignet sind Polyglycolether, insbesondere cyclische, wie z.B. 18-Krone-6 und tertiäre Amine wie z.B. Tributylamin. Bevorzugt sind quarternäre Ammoniumverbindungen wie z.B. Benzyltriethylammoniumchlorid und Tetrabutylammoniumbromid. Geeignet ist aber auch wasserfreies Calciumsulfat.

Die nach den oben genannten Verfahrensvarianten hergestellten erfindungsgemäßen Verbindungen können nach den üblichen Methoden aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion. Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb-und geruchlose Flüssigkeiten sowie Kristalle dar, die schwerlöslich in Wasser, bedingt löslich in aliphatischen Kohlenwasserstoffen wie Petrolether, Hexan, Pentan und Cyclohexan, gut löslich in halogenierten Kohlenwasserstoffen wie Chloroform, Methylenchlorid und Tetrachlorkohlenstoff, aromatischen Kohlenwasserstoffen wie Benzol, Toluol und Xylol, Ethern wie Diethylether, Tetrahydrofuran und Dioxan, Carbonsäurenitrilen wie Acetonitril, Alkoholen wie Methanol und Ethanol, Carbonsäureamiden wie Dimethylformamid und Sulfoxiden wie Dimethylsulfoxid, sind.

Die Verbindungen der allgemeinen Formel I besitzen ein asymmetrisches Kohlenstoffatom $C^*$, wenn $R_1$ und $R_2$ nicht identisch sind. Bei der Synthese fallen die Endprodukte normalerweise als racemisches Gemisch an und können in bekannter Weise in die optischen Antipoden gespalten werden. Die einzelnen Enantiomeren können auch durch chemische Synthese dargestellt werden, wenn man chirale Verbindungen der Formel III verwendet. Im Verlauf der Umsetzung nach den Reaktionsvarianten (a) oder (b) findet eine Walden'sche Umkehr statt, so daß man zur Herstellung der R-konfigurierten Verbindungen der Formel I ein Ausgangsmaterial III verwenden muß, welches am asymmetrischen Kohlenstoffatom die S-Konfiguration besitzt.

Die Ausgangsverbindungen zur Herstellung der erfindungsgemäßen Verbindungen sind an sich bekannt oder können nach an sich bekannten Verfahren hergestellt werden (siehe z.B. Chem. Pharm. Bull. Japan - (1961) 9, 970).

Die erfindungsgemäßen Verbindungen zeichnen sich durch eine allgemeine, nicht nur spezifisch gegen Gräser gerichtete herbizide Wirkung aus, worin sie überraschenderweise bekannten konstitutionsanalogen Wirkstoffen überlegen sind.

Von besonderem Vorteil ist es, daß diese Verbindungen in Aufwandmengen von etwa 0,1 kg Wirkstoff/ha das Aufkommen von einkeim-und zweikeimblättrigen Unkräutern in Nutzpflanzkulturen hemmen oder sogar verhindern, ohne die Kulturen bis zu Aufwandmengen von etwa 5 kg Wirkstoff/ha zu schädigen.

Unkräuter dieser Art sind zum Beispiel Viola, Galium, Veronica, Centaurea, Ipomoea, Abutilon, Sesbania, Datura, Chrysanthemum, Polygonum, Sida, Xanthium, Amaranthus, Setaria, Digitaria, Echinochloa und Alopecurus, die sich demzufolge in vorteilhafter Weise im Nachauflaufverfahren in Kulturen wie Weizen, Gerste, Roggen, Hafer, Mais, Reis, Soja und Baumwolle selektiv bekämpfen lassen.

Die erfindungsgemäßen Verbindungen eignen sich in Abhängigkeit von der Konzentration in der entsprechenden Zubereitung zur Totalunkrautbekämpfung, wie z.B. auf Industrie-und Gleisanlagen und auf Wegen und Plätzen mit und ohne Baumbewuchs. Ebenso können sie zur Unkrautbekämpfung in Dauerkulturen, wie z.B. in Forst-, Ziergehölz-, Obst-, Wein-, Citrus-, Hopfenanlagen und zur selektiven Unkrautbekämpfung in einjährigen Kulturen eingesetzt werden.

Es sei erwähnt, daß einige Wirkstoffe auch für die Desiccation, die Defoliation und als Krautabtötungsmittel geeignet sind.

Die Aufwandmengen für die herbizide Wirkung liegen je nach Anwendungsziel im allgemeinen zwischen 0,05 und 5 kg Wirkstoff/ha. Es können jedoch gegebenenfalls auch höhere Aufwandmengen eingesetzt werden.

Die Anwendungszeit richtet sich nach dem Anwendungsziel und den klimatischen Bedingungen.

Die erfindungsgemäßen Verbindungen können entweder allein, in Mischung miteinander oder mit anderen Wirkstoffen ange wendet werden. Gegebenenfalls können andere Pflanzenschutz-oder Schädlingsbekämpfungsmittel je nach dem gewünschten Zweck zugesetzt werden.

Sofern eine Verbreiterung des Wirkungsspektrums beabsichtigt ist, können auch andere Biozide zugesetzt werden.

Beispielsweise eignen sich als herbizid wirksame Mischungspartner diejenigen Wirkstoffe, die in Weed Abstracts, Vol. 34, No. 3, 1985, unter dem Titel "List of common names and abbreviations employed for currently used herbicides and plant growth regulators" aufgeführt sind.

Außerdem können auch nicht phytotoxische Mittel angewendet werden, die mit Herbiziden und/oder Wuchsregulatoren eine synergistische Wirkungssteigerung ergeben können, wie unter anderem Netzmittel, Emulgatoren, Lösungsmittel und ölige Zusätze.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acylphosphatidylethanolaminen, Phosphatidylinosit, Phosphatidylserin, Lysolecithin und Phosphatidylglycerol.

Zweckmäßigerweise werden die erfindungsgemäßen Wirkstoffe in Form von Zubereitungen wie Pulvern, Streumitteln, Granulaten oder Lösungen unter Zusatz von flüssigen und/oder festen Trägerstoffen beziehungsweise Verdünnungsmitteln und gegebenenfalls Netz-und/oder Haftmitteln angewandt.

Geeignete flüssige Trägerstoffe sind zum Beispiel Wasser, Methanol, Ethanol, Dimethylformamid oder Dimethylsulfoxid. Als feste Trägerstoffe eignen sich Mineralerden, zum Beispiel Tonsil, Silicagel, Talkum oder Kaolin.

An oberflächenaktiven Stoffen sind zu nennen: Kationische, anionische und nicht-ionische Tenside wie zum Beispiel Calciumligninsulfonat, Polyäthylenalkylphenylether, Naphthalinsulfonsäuren und deren Salze, Formaldehydkondensate, Fettalkoholsulfate sowie substituierte Benzolsulfonsäuren und deren Salze.

Der Anteil des oder der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozente flüssige oder feste Trägerstoffe sowie gegebenenfalls biz zu 40 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 1000 l/ha.

Eine Anwendung der Mittel in sogenannten "Low-Volume" oder "Ultra-Low-Volume-Verfahren" ist ebenso möglich wie ihre Applikation in Form von sogenannten Mikrogranulaten.

Zur Herstellung der Zubereitungen werden zum Beispiel die folgenden Bestandteile eingesetzt:

A. SPRITZPULVER

A1) 40 Gewichtsprozent Wirkstoff
25 Gewichtsprozent Tonmineralien
20 Gewichtsprozent gefällte Kieselsäure
15 Gewichtsprozent oberflächenaktive Stoffe auf Basis einer Mischung des Calciumsalzes der Ligninsulfonsäure mit Alkylphenolpolyglycolethern

A2) 25 Gewichtsprozent Wirkstoff
60 Gewichtsprozent Kaolin
10 Gewichtsprozent Kieselsäure
5 Gewichtsprozent oberflächenaktive Stoffe auf Basis des Natriumsalzes des N-Methyl-N-oleyl-taurins und des Calciumsalzes der Ligninsulfonsäure

A3) 10 Gewichtsprozent Wirkstoff
60 Gewichtsprozent Tonmineralien
Gewichtsprozent Kieselsäure
15 Gewichtsprozent oberflächenaktive Stoffe auf Basis des Natriumsalzes des N-Methyl-N-oleyl-taurins und des Calciumsalzes der Ligninsulfonsäure

B. PASTE

45 Gewichtsprozent Wirkstoff
5 Gewichtsprozent Natriumaluminiumsilikat
15 Gewichtsprozent Cetylpolyglycolether mit 8 Prozent Ethylenoxid
2 Gewichtsprozent Spindelöl
10 Gewichtsprozent Polyethylenglycol
23 Teile Wasser

C. EMULSIONSKONZENTRAT

25 Gewichtsprozent Wirkstoff
15 Gewichtsprozent Cyclohexanon
55 Gewichtsprozent Xylol
5 Gewichtsprozent Mischung von Nonylphenylpolyoxyethylen oder Calciumdodecylbenzolsulfonat

Die folgenden Beispiele erläutern die Herstellung der erfindungsgemäßen Verbindungen.

Beispiel 1 (Variante a)

2-[7-(2-Chlor-4-trifluormethylphenoxy)-2-chinolyloxy]propansäuremethylester

7,5 g 7-(2-Chlor-4-trifluormethylphenoxy)-chinolin-2-ol, Hydrobromid werden in 70 ml Dimethylformamid gelöst. Es werden 5,14 g gepulvertes Kaliumcarbonat zugegeben und 15 Minuten bei 60 °C gerührt. Anschließend wird bei dieser Temperatur eine Lösung von 3,69 g 2-Brompropansäuremethylester in 30 ml Dimethylformamid (DMF) zugetropft und 2 Stunden bei 60 °C gerührt. Das DMF wird im Vakuum abgezogen, der Rückstand in Essigsäureethylester aufgenommen und mehrfach mit Wasser gewaschen. Die organische Phase wird mit Magnesiumsulfat getrocknet, eingeengt und an Kieselgel chromatographiert. (Essigsäureethylester/Hexan 1:9).
Ausbeute: 6,2 g = 80,8% d. Th.
$R_f$-Wert: in $CH_2Cl_2$/MeOH 95:5 = 0,82
$n_D$ = 1,5574

Beispiel 2

2-(R)-[7-(2-Chlor-4-trifluormethylphenoxy)-2-chinolyloxy]propansäuremethylester

10 g 7-(2-Chlor-4-trifluormethylphenoxy)-chinolin-2-ol, Hydrobromid werden in 100 ml Dimethylsulfoxid - (DMSO) gelöst und mit 8,4 g Kaliumcarbonat und 7 g wasserfreiem Calciumsulfat (Sikkon) über Nacht bei Raumtemperatur gerührt. Anschließend wird bei Raumtemperatur eine Lösung von 7,6 g 2-(s)-(p-Tolylsulfonyloxy)-propansäuremethylester in 30 ml DMSO zugetropft und 4 Stunden bei Raumtemperatur gerührt - (DC-Kontrolle). Es wird über Kieselgur abgesaugt und das Filtrat mehrfach mit Ether extrahiert. Die vereinigten Etherphasen werden mit NaCl-Lösung gewaschen, über $MgSO_4$ getrocknet und eingedampft. Säulenchromatographie an Kieselgel mit Hexan/Essigester 9:1 ergibt 2-(R)-[7-(2-Chlor-4-trifluormethylphenoxy)-2-chinolyloxy]-propansäuremethylester.
Ausbeute: 6 g = 59% d. Th.
n = 1,5479
$\alpha$ = + 18,5 °

<u>Beispiel 3</u> (Variante b)

2-[7-(2-Chlor-4-trifluormethylphenoxy)-2-chinolyloxy]propansäureisopropylester

7,5 g 7-(2-Chlor-4-trifluormethylphenoxy)-chinolin-2-ol, Hydrobromid werden in 70 ml Dimethylformamid gelöst. Es werden 2,16 g Natriumhydrid (80 %ig in Öl) zugegeben und 15 Minuten bei Raumtemperatur gerührt. Anschließend wird bei dieser Temperatur eine Lösung von 3,51 g 2-Brompropansäureisopropylester in 30 ml DMF zugetropft und 4 Stunden bei 60 °C gerührt. Das DMF wird im Vakuum abgezogen, der Rückstand in Essigsäureethylester aufgenommen und mehrfach mit Wasser gewaschen. Die organische Phase wird mit $MgSO_4$ getrocknet, eingeengt und an Kieselgel chromatographiert - (Essigsäureethylester/Hexan 1:9).
Ausbeute: 5,14 g = 63 % d. Th.
$R_f$-Wert in $CH_2Cl_2$/MeOH 95:5 = 0,84
Fp.: 119 C

Herstellung des Ausgangsmaterials

7-(2-Chlor-4-trifluormethylphenoxy)-chinolin-2-ol, Hydrobromid

34,17 g 7-(2-Chlor-4-trifluormethylphenoxy)-1,2,3,4-tetrahydrochinolin-2-on werden in 1300 ml trockenem Tetrachlorkohlenstoff in der Siedehitze gelöst. Die Lösung wird mit einer 300 W Lampe bestrahlt und 16,74 g getrocknetes Brom in 100 ml trockenem Tetrachlorkohlenstoff werden langsam in der Siedehitze zugetropft. Nachdem man ca. 5,0 ml der Bromslösung zugetropft hat, fallen weiße Kristalle aus, die man nach beendeter Bromzugabe und Abkühlen der Suspension auf Raumtemperatur absaugt und mit Pentan

nachwäscht.
Ausbeute: 41,27 g = 98 % d. Th.
Fp.: 225-228 °C
$R_f$-Wert: 0,12 in $CH_2Cl_2$/MeOH 95:5

7-(2-Chlor-4-trifluormethylphenoxy)-1,2,3,4-tetrahydrochinolin-2-on

35 g 7-Hydroxy-1,2,3,4-tetrahydro-2-chinolon werden in 500 ml Dimethylsulfoxid gelöst. Es wird auf 160 °C Innentemperatur aufgeheizt und bei dieser Temperatur werden 46,02 g 3,4-Dichlorbenzotrifluorid dazugetropft. Nach 1 Stunde bei 160 °C (DC-Kontrolle) wird auf Raumtemperatur abgekühlt und die Lösung in 2500 ml Eiswasser gegossen. Das ausgefallene Produkt wird abgesaugt, mit wenig Wasser gewaschen und getrocknet. Anschließend wird in Essigester/Hexan umkristallisiert.
Ausbeute: 34,4 g = 47,94 % d. Th.
Fp.: 194-195 °C
$R_f$-Wert: 0,50 in $CH_2Cl_2$/MeOH 95:5

In analoger Weise werden die folgenden Verbindungen hergestellt

| Nr. | Verbindung | Reaktions-variante | Phsysikal. Konstante |
|---|---|---|---|
| 4 | 2-(R)-[7-(2-Chlor-4-trifluor-methylphenoxy)-2-chinolyloxy]-propansäureethylester | a | Fp. 98-99 °C<br>$\alpha_D$ = +34,9 ° |
| 5 | 2-(S)-[7-(2-Chlor-4-trifluor-methylphenoxy)-2-chinolyloxy]-propansäureethylester | a | Fp. 90-91 °C<br>αD = -36,4 ° |
| 6 | 2-[7-(2-Chlor-4-trifluormethyl-phenoxy)-2-chinolyloxy]-propan-säureethylester (racemisch) | a | Fp. 98 °C |

| Nr. | Verbindung | Reaktions-variante | Physikal. Konstante |
|---|---|---|---|
| 7 | 2-/7-(2-Chlor-4-trifluormethyl-phenoxy)-2-chinolyloxy7-propansäure-2,2,2-trifluorethylester | a | Fp. 108-10 °C |
| 8 | 2-/7-(2-Chlor-4-trifluormethyl-phenoxy)-2-chinolyloxy7-propansäure-n-butylester | a | Fp. 63-64 °C |
| 9 | 2-/7-(2-Chlor-4-trifluormethylphen-oxy)-2-chinolyloxy7-propansäure-2-propinylester | a | Fp. 81-83 °C |
| 10 | 2-/7-(2-Chlor-4-trifluormethylphen-oxy)-2-chinolyloxy7-propansäure-methoxyethylester | a | $n_D^{20}$= 1.5522 |
| 11 | 2-/7-(2-Chlor-4-trifluormethylphen-oxy)-2-chinolyloxy7-propansäure | a | Fp. 132-34 °C |
| 12 | 2-/7-(2-Chlor-4-trifluormethylphen-oxy)-2-chinolyloxy7-propansäure, Natriumsalz | a | Fp. 203 °C |
| 13 | 2-/7-(2-Chlor-4-trifluormethylphen-oxy)-2-chinolyloxy7-propansäure-n-propylester | a | Fp. 92-94 °C |
| 14 | 2-/7-(2-Chlor-4-trifluormethylphen-oxy)-2-chinolyloxy7-propansäure-2-propenylester | a | Fp. 87-89 °C |
| 15 | 2-/7-(2-Chlor-4-trifluormethylphen-oxy)-2-chinolyloxy7-propansäure-2-ethoxyethylester | c | $n_D^{20}$= 1,5378 |

| Nr. | Verbindung | Reaktions-variante | Physikal. Konstante |
|---|---|---|---|
| 16 | 2-[7-(2-Chlor-4-trifluormethyl-phenoxy)-2-chinolyloxy]-propan-säurefurfurylester | a | $n_D^{20}$= 1,5578 |
| 17 | 2-[7-(3-Chlor-5-trifluormethyl-2-pyridyloxy)-2-chinolyloxy]-propan-säuremethylester | a | Fp. 78-77 °C |
| 18 | 2-[7-(2-Chlor-4-trifluormethyl-phenoxy)-2-chinolyloxy]-3-methyl-butansäureethylester | a | $n_D^{20}$= 1,5438 |
| 19 | 2-[7-(2-Chlor-4-trifluormethyl-phenoxy)-2-chinolyloxy]-essigsäure-ethylester | a | Fp. 88-90 °C |
| 20 | 2-[7-(2-Chlor-4-trifluormethyl-phenoxy)-2-chinolyloxy]-butansäure-ethylester | a | $n_D^{20}$= 1,5458 |
| 21 | 2-[7-(2-Chlor-4-trifluormethyl-phenoxy)-2-chinolyloxy]- propan-säure-2-butylester | a | Fp:69-71°C |
| 22 | 2-[7-(2-Chlor-4-trifluormethyl-phenoxy)-2-chinolyloxy] -2-methylpropansäureethylester | a | $n_D^{20}$= 1,5358 |
| 23 | 2-[7-(2-Chlor-4-trifluormethyl-phenoxy)-2-chinoxyloxy]-propan-säureethylamid | a | Fp: 118°C |
| 24 | 2-[7-(2-Chlor-4-trifluormethyl-phenoxy) -2-chinolyloxy] -propan säureamid | a | Fp:139°C |

Die nachfolgenden Beispiele dienen zur Erläuterung der Anwendungsmöglichkeit der erfindungsgemäßen Verbindungen in Form ihrer Zubereitungen.

Beispiel A

Im Gewächshaus wurden die erfindungsgemäßen Verbindungen in einer Aufwandmenge von 3,0 kg Wirkstoff/ha emulgiert in 500 Litern Wasser/ha, auf Brassica napus und Chrysanthenum segetum als Testpflanzen im Nachauflaufverfahren gespritzt. 3 Wochen nach der Behandlung wurde das Behandlungsergebnis bonitiert, wobei 0 = keine Wirkung und 4 = Vernichtung der Pflanzen bedeutet.

Mit den erfindungsmäßen Verbindungen gemäß den Beispielen 1 bis 4 und 5 bis 24 wurde eine völlige Vernichtung (= 4) der Testpflanzen erreicht.

Beispiel B

Im Gewächshaus wurden die in der Tabelle aufgeführten Pflanzen nach dem Auflaufen mit den aufgeführten Verbindungen in einer Aufwandmenge von 0,3 kg Wirkstoff/ha behandelt. Die Verbindungen wurden zu diesem Zweck als Emulsion mit 500 Litern Wasser/ha gleichmäßig über die Pflanzen versprüht. Hier zeigte 3 Wochen nach der Behandlung die erfindungsgemäße Verbindung eine hohe Selektivität bei ausgezeichneter Wirkung gegen das Unkraut. Das Vergleichsmittel zeigte diese Wirkung nicht in gleichem Maße.

| | Solanum sp. | Brassica sp. | Viola sp. | Stellaria sp. | Chrysanthemum sp. | Helianthus sp. | Ipomoea sp. | Abutilon sp. | Sorghum sp. | Setaria sp. | Oryzae sativa | Triticum aestivum | Hordeum distichum |
|---|---|---|---|---|---|---|---|---|---|---|---|---|---|
| Erfindungsgemäßes Mittel gemäß Beispiel 6 | 4 | 4 | 4 | 4 | 4 | 4 | 4 | 4 | 3 | 0 | 0 | 0 | 3 |
| Vergleichsmittel Acifluorfen | 4 | 4 | 4 | 2 | 4 | 3 | 4 | 3 | 3 | 4 | 1 | 3 | 3 |

**Ansprüche**

1. 7-(Aryloxy)-2-chinolyloxy-alkancarbonsäurederivate der allgemeinen Formel I

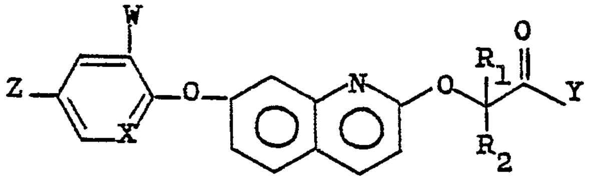 (I),

in welcher
Z und W unabhängig voneinander Wasserstoff, Halogen, einen $C_{1-4}$-Alkylrest, einen Trihalogenmethylrest oder eine Cyanogruppe bezeichnen,
X eine CH-Gruppe, eine C-Halogen-Gruppe oder ein Stickstoffatom bedeutet,
$R_1$ und $R_2$ unabhängig voneinander Wasserstoff oder eine $C_{1-4}$-Alkylgruppe darstellen und
Y die Gruppe

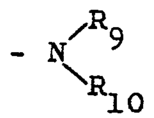

bedeutet,
wobei $R_9$ und $R_{10}$ gleich oder verschieden sind und jeweils Wasserstoff, einen $C_{1-18}$-Alkylrest, einen substituierten $C_{1-18}$-Alkylrest, einen $C_{2-8}$-Alkenyl-oder Alkinylrest, einen gegebenenfalls substituierten Aryl-$C_{1-3}$-Alkylrest, einen gegebenenfalls substituierten $C_{3-8}$-cycloaliphatischen Kohlenwasserstoffrest, einen gegebenenfalls substituierten $C_{3-8}$-Cycloalkyl-$C_{1-3}$-alkylrest, einen gegebenenfalls ein-oder mehrfach durch $C_{1-6}$-Alkyl und/oder Halogen und/oder $C_{1-6}$-Alkoxy und/oder die Nitrogruppe und/oder die Trifluormethylgruppe substituierten aromatischen Kohlenwasserstoffrest oder $R_9$ und $R_{10}$ gemeinsam mit dem N-Atom die Morpholino-, Piperidino-oder Pyrrolidinogruppe darstellen oder
Y den Rest $UR_3$ bezeichnet, wobei U ein Sauerstoff-oder Schwefelatom bedeutet und
$R_3$ einen $C_{1-18}$-Alkylrest, der gegebenenfalls durch Halogen oder Cyano substituiert oder durch Sauerstoff oder Schwefel ein-oder mehrfach unterbrochen ist, einen $C_{3-12}$-Alkenyl-, $C_{3-12}$ -Alkinyl oder $C_{3-12}$-Cycloalkylrest, einen Phenyl-oder Benzylrest, der unsubstituiert oder durch Halogen, $C_{1-4}$-Alkyl, $C_{1-4}$-Alkoxy, $C_{1-4}$-Alkylthio, Nitro, Cyano oder Trifluormethyl ein-oder mehrfach substituiert ist oder einen 5-6 gliedrigen heterocyclischen Rest darstellt;
Wasserstoff, Lithium, Natrium oder Kalium oder ein Äquivalent von Zink, Mangan , Calcium, Magnesium oder Barium oder ein Ammonium-Ion der Formel

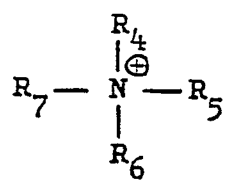

bedeutet, wobei
$R_4$, $R_5$, $R_6$ und $R_7$ unabhängig voneinander Wasserstoff, Alkyl mit bis zu 6 Kohlenstoffatomen oder halogen-, hydroxy-oder alkoxy-substituiertes Alkyl mit bis zu 6 Kohlenstoffatomen oder eine Carbalkoxyalkyl-Gruppe -$(CH_2)_nCOOR_8$ mit $n = 1\text{-}4$ und $R_8 = C_{1-4}$-Alkyl darstellen.

2. 7-(Aryloxy)-2-chinolyloxy-alkancarbonsäurederivate der Formel I gemäß Anspruch 1, wobei am asymmetrischen C-Atom die R-Konfiguration vorliegt.

3. 7-(Aryloxy)-2-chinolyloxy-alkancarbonsäurederivate gemäß Anspruch 1 der allgemeinen Formel I, in welcher
Z und W unabhängig voneinander ein Fluor-, Chlor-, Brom-oder Wasserstoffatom, eine Cyano-, eine Trifluormethyl-, eine Trichlormethylgruppe, eine Methyl-, Ethyl-oder Isopropylgruppe bedeuten,
X eine CH-Gruppe, eine C-Fluor-, eine C-Chlor-, eine C-Brom-Gruppierung oder ein Stickstoffatom bedeutet,
$R_1$ und $R_2$ unabhängig voneinander Wasserstoff oder einen gegebenenfalls durch Chlor oder Brom eine-oder mehrfach substituiertes Methyl, Ethyl, n-Propyl, n-Butyl, iso-Propyl sowie Fluormethyl und Difluormethyl bedeuten,
Y den Rest $UR_3$ bezeichnet, wobei U ein Sauerstoff-oder Schwefelatom ist und $R_3$ Wasserstoff, Methyl, Ethyl, Propyl, Isopropyl, Butyl, Allyl, Propargyl, Cyclohexyl, Pentyl, Hexyl, Dodecyl, Methoxyethyl, Ethoxyethyl, Benzyl, 4-Chlorbenzyl, 4-Nitrobenzyl, Phenyl, 4-Chlorphenyl, 4-Nitrophenyl, 3-Phenoxybenzyl oder Lithium, Natrium oder Kalium oder ein Äquivalent von Zink, Mangan, Calcium, Magnesium oder Barium oder Ethylammoniumion, Diethylammoniumion, Triethylammoniumion, Tetraethylammoniumion oder Tetrabutylammoniumion bedeutet
oder
die Gruppe

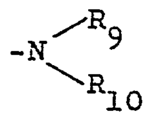

darstellt, wobei $R_9$ und $R_{10}$ unabhängig voneinander Wasserstoff, Methyl, Ethyl, Propyl, Isopropyl, n-Butyl, tert.-Butyl, 2,2-Dimethyl-1-propyl, n-Heptyl, n-Nonyl, n-Undecyl, n-Octadecyl, 3-Methylbutyl, 4-Methyl-2-pentyl, Isobutyl, 3,3-Dimethylbutyl, 2-Chlorethyl, 3-Chlorpropyl, 3-Brompropyl, 2-Bromethyl, 1-Phenoxy-2-propyl, Tetrahydrofurfuryl, Ethoxycarbonylmethyl, Cyanmethyl, 2,2-Dimethoxyethyl oder 2-Ethoxyethyl, Cyclohexylmethyl, 4-Cyanocyclohexylmethyl, 4-Hydroxymethylcyclohexylmethyl, Cycloheptylmethyl oder Cyclooctylmethyl oder Cyclopropylmethyl, 2-Propenyl, 2-Butenyl, 2-Methyl-2-propenyl, 2-Propinyl oder 3-Ethyl-1-pentin-3-yl, Benzyl, 4-Chlorbenzyl, 3-Chlorbenzyl, 2-Chlorbenzyl, 4-Fluorbenzyl, 3-Fluorbenzyl, 2-Fluorbenzyl, 4-Methylbenzyl, 3-Methylbenzyl, 2-Methylbenzyl, 3,4-Methylendioxybenzyl, 2,4-Dichlorbenzyl, 3,4-Dichlorbenzyl, 4-Methoxybenzyl, 3-Methoxybenzyl, 2-Methoxybenzyl, 2-Pyridylmethyl, 3-Pyridylmethyl, 4-Pyridylmethyl, $\alpha,\alpha$-Dimethylbenzyl, 1-Phenylethyl, 2-Phenylethyl, 1,2-Diphenylethyl, 2,2-Diphenylethyl, 4-Fluor-$\alpha$-methylbenzyl, 3-Phenylpropyl, Cyclopropyl, Cyclopentyl, Cyclohexyl, 2-Methylcyclohexyl, 3-Methylcyclohexyl, 4-Methylcyclohexyl, 1-Ethinylcyclohexyl, Cycloheptyl oder Cyclooctyl, Phenyl, 3-Chlorphenyl, 2-Chlorphenyl, 4-Chlorphenyl, 2,4-Dichlorphenyl, 4-Fluorphenyl, 2-Methylphenyl, 3-Methylphenyl, 4-Methylphenyl, 1-Naphthyl, 2-Methoxyphenyl, 3-Methoxyphenyl oder 4-Nitrophenyl bedeuten.

4. Verfahren zur Herstellung von Verbindungen der Formel I, dadurch gekennzeichnet, daß man

a) Verbindungen der allgemeinen Formel II

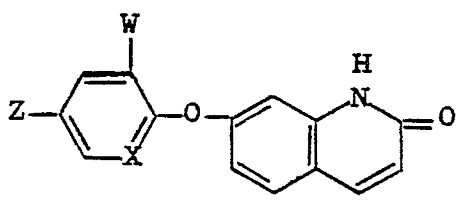

(II),

in der Z, W und X die bei Formel I angegebene Bedeutung haben,
mit einer Verbindung der allgemeinen Formel III

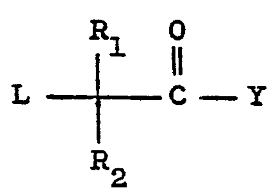

(III),

in der $R_1$, $R_2$ und Y die in Formel I angegebene Bedeutung haben und L für ein Halogenatom, den Mesyl- oder Tosyl-Rest steht, umsetzt
oder

b) eine Verbindung der allgemeinen Formel IV

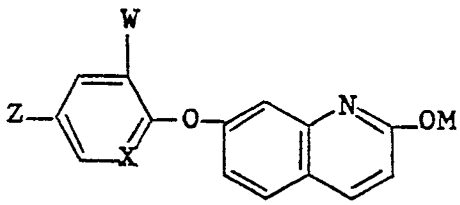

(IV),

in der Z, W und X die in Formel I angegebene Bedeutung haben und M ein Alkalimetall bezeichnet, mit einer Verbindung der Formel III

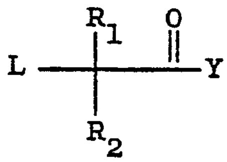

(III),

in der $R_1$, $R_2$, L und Y die oben angegebene Bedeutung haben, umsetzt, oder

c) eine Verbindung der allgemeinen Formel V

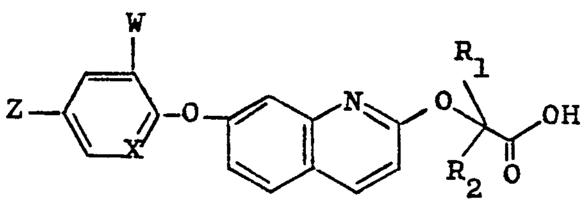

(V),

in der Z, W, X, $R_1$ und $R_2$ die in Formel I angegebene Bedeutung haben, mit einem Reagenz umsetzt, welches die Carboxylgruppe gegenüber einem nucleophilen Angriff aktiviert, vorzugsweise mit Thionylchlorid, Dicyclohexylcarbodiimid oder Carbonyldiimidazol, und das so erhaltene aktivierte Carbonsäurederivat mit einer Verbindung der Formel VI
H -Y (VI),
in der Y die in Formel I angegebene Bedeutung hat, reagieren läßt.

5. Mittel mit herbizider Wirkung gekennzeichnet durch einen Gehalt an mindestens einer Verbindung gemäß den Ansprüchen 1 bis 3.

6. Mittel gemäß Anspruch 5 in Mischung mit Träger-und/oder Hilfsstoffen.

7. Mittel gemäß Anspruch 5, hergestellt nach Verfahren gemäß Anspruch 4.

8. Verwendung der Verbindungen gemäß den Ansprüchen 1 bis 3 zur Bekämpfung dikotyler und monokotyler Pflanzenarten in Nutzpflanzenkulturen.

Europäisches Patentamt

# EUROPÄISCHER RECHERCHENBERICHT

Nummer der Anmeldung: EP 86115115.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | DE - A1 - 3 101 544 (HOECHST)<br>* Ansprüche 1,2,3,4 *<br>-- | 1,4,5,8 | C 07 D 215/22<br>C 07 D 405/12<br>C 07 D 401/12<br>A 01 N 43/42 |
| A | DE - A1 - 3 337 044 (BASF)<br>* Ansprüche 1,3,4,5 *<br>---- | 1,5,6,8 | |
| | | | RECHERCHIERTE SACHGEBIETE (Int. Cl.4) |
| | | | C 07 D 215/00<br>C 07 D 401/00<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 10-02-1987 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82